# EUROPEAN PATENT SPECIFICATION

(11) **EP 2 298 286 B1**
(45) Date of publication and mention of the grant of the patent: **30.11.2016**
(21) Application number: 09170634.1
(22) Date of filing: 18.09.2009
(51) Int. Cl.: A61K 9/16, B01J 2/02, F26B 3/12

(54) **Process for dewatering of product powders**
Verfahren zur Entwässerung von Produktpulvern
Procédé de déshydratation de poudres de produit

(43) Date of publication of application: 23.03.2011
(73) Proprietor: Messer Group GmbH, 65812 Bad Soden (DE)
(72) Inventor: Galli, Leonardo, 50065, Pontassieve (IT); Esposito, Pierandrea, 08017, Barcelona (ES); Segale, Lorena, 27029, Vigevano (IT); Giovannelli, Lorella, 28100, Novara (IT); Pattarino, Franco, 10126, Torino (IT); Danan, Hana, 08017, Barcelona (ES)
(74) Representative: Münzel, Joachim R.

(56) References cited:
- EP-A1- 0 943 326
- EP-A1- 1 027 144
- EP-B1- 1 077 691
- WO-A1-2005/049192
- WO-A1-2006/101352
- WO-A1-2007/024133
- WO-A1-2007/097626
- WO-A2-03/087335
- WO-A2-2004/058156
- WO-A2-2006/053882
- WO-A2-2008/033023
- US-A- 5 851 453
- US-B1- 6 294 196
- "SURFACE-MODIFIED PARTICLES OF IBANDRONATE" IP.COM JOURNAL, IP.COM INC., WEST HENRIETTA, NY, US, 3 April 2009 (2009-04-03), XP013130816 ISSN: 1533-0001

## Description

The invention relates to a process for the production of dry products in powder form and to pharmaceutical compositions produced according to such process.

Known methods for the simultaneous dewatering and pulverisation of products make use of the so-called dry spraying processes. In those processes, water-containing dispersions such as emulsions are sprayed within a spray tower into which at the same time a hot gas at a temperature of 140°C - 300°C is fed in counter flow to the sprayed dispersion. Due to the high temperatures, the water is removed, at least in part, from the product. However, these processes cannot be used for drying products which are very sensitive to heat.

In WO 2007/097626 A1 a process is described in which a water-containing product, in particular an W/O or O/W - emulsion with a water content of 10 - 50 weight-%, is brought into contact with a supercritical fluid while it is sprayed within a spray tower. The supercritical fluid used in this process is carbon dioxide or nitric oxide. During the process, the water content of the product is dissolved in the supercritical fluid and thus, the remaining product is dried. Therefore, it is essentially for this process that during the whole treatment the fluid is kept in the supercritical state, i.e. at a temperature and a pressure well above the temperature and the pressure of the critical point of the fluid. For example, the temperature and pressure within the spraying tower is about 5 - 20 K and 50 - 75 bar *above* the respective values of the critical point of the supercritical fluid. At a consequence, this process cannot be used for substances which are sensitive to high pressure.

In WO 2006//053882 a process comprising an atomisation step is described in which an emulsion is sprayed through a nebulizer or a capillary nozzle in the presence of liquid carbon dioxide which sprayed through a second nozzle.

The object of the present invention is to provide a process for drying and spraying substances into small micro particles that works without high temperatures or pressurisations. Another object of the invention is to provide pharmaceutical compositions consisting of small micro particles containing heat and/or pressure sensitive substances.

This object is achieved by a process described in patent claim 1. Advantageous embodiments of the invention are described in the depending claims.

The process for production of dry powdery products according to the invention therefore is characterized in that a feed substance or a mixture of feed substances is mixed with water forming a water-containing mixture, the water-containing mixture is brought into contact at a two-component nozzle with a supercritical or liquid pressurized medium, and the supercritical or liquid pressurized medium is released, whereby an atomisation and drying of the mixture is achieved.

The invention makes use of a known method for spraying and cooling of particles, described in EP 1 027 144 A1. According to this process, the substance to be atomized is liquefied and mixed with or dissolved in a pressurized fluid in liquid or supercritical state, e.g. liquid or supercritical carbon dioxide. The mixture of liquid or supercritical carbon dioxide and the substance then is fed to a nozzle within a spraying chamber. Alternatively, both substances are fed simultaneously to the orifice of a two-component nozzle. Within the spraying chamber, the pressure is much lower than the pressure of the liquefied or supercritical carbon dioxide; therefore the medium is depressurized and cooled down, forming gaseous carbon dioxide and carbon dioxide snow, whereby the latter is sublimating rapidly. Due to the contact between the substance and the depressurizing medium during the spraying process, the substance is atomized and cooled down, yielding a fine cold powder.

Surprisingly, it has now been found that this process can also be used as a very effective way for drying water-containing mixtures. By spraying of water-containing mixtures using this process the water content of the mixture can be reduced by more than 85-90%, in some cases by more than 98%. The drying process occurs when the water containing substance is brought into contact with a *releasing* pressurized liquefied or supercritical medium. Thereby, the drying process takes place without applying high temperatures or high pressures (equal or even above the critical pressure of the medium) to the substance itself.

A "water-containing mixture", within the context of the present invention, shall be any type of dispersion, emulsion, double emulsion, microemulsion, suspension, colloid, or solution of any kind. For example, the water-containing mixture can be an emulsion of a water-rich phase in an oil-matrix, in which water-rich phase a hydrophilic active agent can be dissolved or dispersed. The water content of the water-containing mixture can be up to 50 weight-% or even more; however, a water content of less than 20-30 weight-% is preferred. The water-containing mixture is generally in liquid state or has a low viscosity. However, by heating to a temperature above the respective melting point, it may also be possible to use as water-containing mixture substances which are solid or of high-viscosity at ambient temperature.

In particular, the water-containing mixture can be a mixture of one or more pharmaceutical active agents and one or more excipients. Depending on the composition of the water-containing mixture before spraying at the two-component nozzle, in particular depending on the kinds and relative amounts of the feed substances, micro particles are formed in the spraying process which consist either of a pure substance or of a mixture - like a solution, a dispersion or an emulsion - having a low water content. In particular in case of spraying a dispersion or emulsion consisting of a hydrophilic and a lipophilic phase, an intimately mixing of the hydrophilic and lipophilic components is achieved within the microparticles, while most or all the water in the initial mixture is removed. The excipients dissolved or dispersed in the water-containing mixture are designed to fulfil a specific function or a combination of functions relevant for the purpose of the pharmaceutical formulation development, e.g. they are designed to stabilize the emulsion, or to enhance the adsorption or solution of poorly soluble drugs.

In a preferred embodiment of the invention, the feed substance or the mixture of feed substances is heated before or during it is conducted to the two component nozzle. This assures that the feed substance or the mixture of feed substances will be in the liquid state when it is sprayed. For example, in the case of some agents and excipients used for pharmaceutical purposes, a preferred temperature of the water containing mixture at the time of spraying is between ca. 60°C and ca. 95°C which is upheld by heating the tubes conducting the water-containing mixture to the nozzle. In another advantageous embodiment of the invention, the feed substance or the mixture of feed substances is pressurized to a pressure of about 2 to 10 bar. Thus, the values of temperatures and pressures used for a proper performance of the invention are well below the temperatures and pressures used in drying processes according to the state of the art.

In particular, the process according to the invention can be used for the production of dry microparticles which consist of or contain water soluble drugs. In this case, preferably at least one of the feed substances is a hydrophilic substance which is dissolved or dispersed in water before it is fed to the two component nozzle. The spraying of such kind of solution or dispersion leads to the formation of a highly concentrated, dry powder consisting of or containing the hydrophilic substance.

In another preferred embodiment of the invention, one or more lipophilic or amphiphilic excipient/s is/are fed to the water or to an aqueous solution or dispersion of an hydrophilic substance and mixed with the latter forming a dispersion or emulsion. After spraying such emulsion or dispersion, dry microparticles are formed in which lipophilic/amphiphilic and hydrophilic components are mixed intimately. Within this intimate mixture the physical status of the respective substances can be amorphous or, partially or totally crystalline. The hydrophilic component can also be molecularly dispersed within the other component(s), as solid or pasty solution or dispersion.

The process according to the invention may also be used for the spraying of water containing mixtures, of which at least one of the feed components is formed of or contains one of the following substances or classes of substances: water soluble salts of pharmaceutically active agents, in particular hydrophilic, water soluble drug molecules such as, e.g. : biphosphonates salts, such as ibendronate, alendronate, zoledronate salts; GABA modulators, like pregabaline or gabapentin; more in general all pharmaceutical substances classified as BCS (Biopharmaceutical Classification) Class I and III -high solubility drugs-, preferably, but not exclusively, Class III (high solubility, low permeability drugs).

The process according to the invention may also be applied to water soluble molecules of proteinaceous nature, such as proteins, peptides, and derivatives such as PEGylated proteins or peptides (PEG: polyethylene glycol).

In particular, the water containing mixture may contain Ibandronate (in particular Sodium Ibandronate) or Metoclopramide. Also advantageous is a feed component containing fatty alcohols (in particular cetearyl alcohol), fatty acids like stearic-palmitic acid or fatty esters such as glyceryl behenate (in particular Compritol^{®} series), and their derivatives as an excipient or a mixture thereof. Polyethylene glycols (PEG), Poloxamers and PEG-fatty acid or lipidic derivatives are also advantageous excipients components for the invention, usable alone or mixed with fatty or polymeric components.

Preferably, the pressurized liquid or supercritical medium is carbon dioxide or nitric oxide.

The process according to the invention is applied preferably in the production of pharmaceutical products.

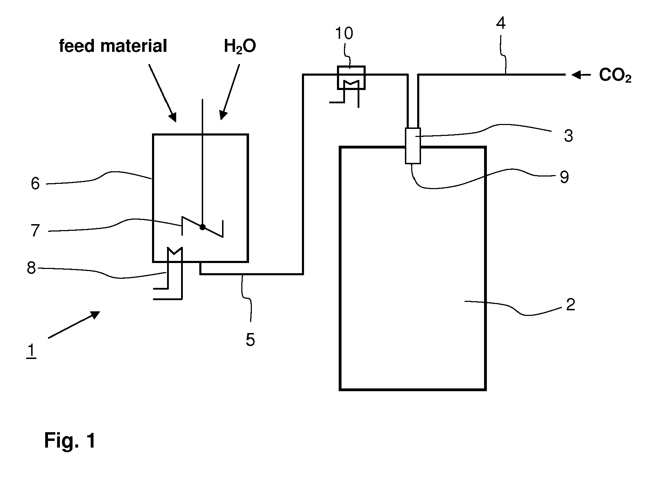

According to the drawing in Fig. 1, a preferred equipment is described which can used for the process according to the invention.

Fig. 1 shows schematically a device 1 for the production of a powder consisting of dry microparticles. The device 1 comprises a pressure vessel 2 in which lower space the produced powder is stored. At the top of vessel 2 a two component nozzle 3 is arranged. Two conducting lines are connected to the nozzle 2: A first line 4 for conducting a pressurized liquid or a supercritical medium, and a second line 5 for conducting a water containing substance. In the example according to Fig. 1, liquid or supercritical carbon dioxide is used as pressurized medium conducted through line 4 at a pressure of about 60-80 bar.

The water containing substance is prepared in a feed vessel 6 which is fluidly connected to line 5. The feed vessel 6 is designed as a pressure-vessel and comprises a mixing device 7 as well as a heater 8.

The water containing mixture prepared in feed vessel 6 consists, for example, of a W/O - emulsion in which a hydrophilic substance dissolved or dispersed in water is arranged within a matrix of an lipophilic or amphiphilic substance. In order to produce the emulsion, the respective feed substances - e.g. active agents like Sodium Ibandronate or Metoclopramide and/or excipients like stearic palmitic acid, cetearyl alcohol, polyethylenglycol (PEG 6000) or Compritol^{®}, are filled into the feed vessel 6 and mixed under a pressure of about 2 to 10 bar and a temperature between 60°C and 96°C, leading to the formation of an emulsion.

During operation of the device 1, the water containing mixture formed in the feed vessel 6 is transported through line 5 to the two component nozzle 3. During the transport, the water containing mixture may also be heated using a heating device 10. Simultaneously the liquid or supercritical carbon dioxide is fed to nozzle 3 through line 4. The two component nozzle 3 can be of the internal mixing type where the components fed through lines 4,5 are mixed within the body of the nozzle before sprayed, or of the external mixing type in which the components are fed to separate orifices arranged distanced from or concentric to each other at the opening of the nozzle 3. In the latter case, the components are mixed in the front of the nozzle within the vessel 2. Leaving the nozzle 3, the liquefied or supercritical carbon dioxide is released and depressurized to the pressure value prevailing inside the vessel 2, which may be e.g. 1 - 2 bar. Due to the expansion of the carbon dioxide, the emulsion will be atomized and cooled down to a temperature well below the melting point of the continuous phase of the emulsion. The carbon dioxide partly passes into its gaseous state and partly into dry ice particles which will sublime rapidly. At the same time, the water content of the sprayed emulsion will remarkably decrease yielding a dry, freely flowable, pourable powder.

### Example 1:

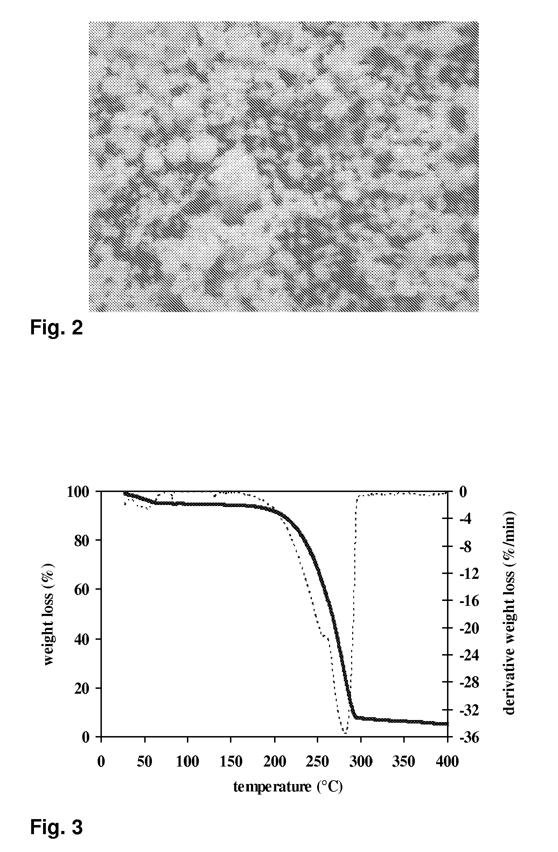

Production of a dry powder consisting of Sodium Ibandronate (Na-IBAN) - containing micro particles:
A concentrated aqueous solution of sodium Ibendronate (Na-IBAN) (about 285 mg/ml) was mixed with molten stearic - palmitic acid (SPA) with a ratio Na IBAN to SPA of 1:9 within the feed vessel at a pressure of 3.5 bar and a temperature of 70°C, forming an emulsion. The water content of the emulsion was ca. 26 weight %. After spraying using liquefied carbon dioxide at a pressure of 60 bar, a dry, pourable micronized powder was formed with a water content of less than 5 weight-%. The microscopic appearance of micronized powder, measured by optical stereomicroscope, is of small, white, opaque, spherical particles (Fig. 2).

A TGA plot (thermogravimetric analysis and first derivative-DTG) (Fig 3) shows that the residual water in the powder is about 5% w/w, as measurable by weight loss between 50 and 100 °C. Water removed by the process was about 81 % of total amount of water originally added to the mixture.

### Example 2:

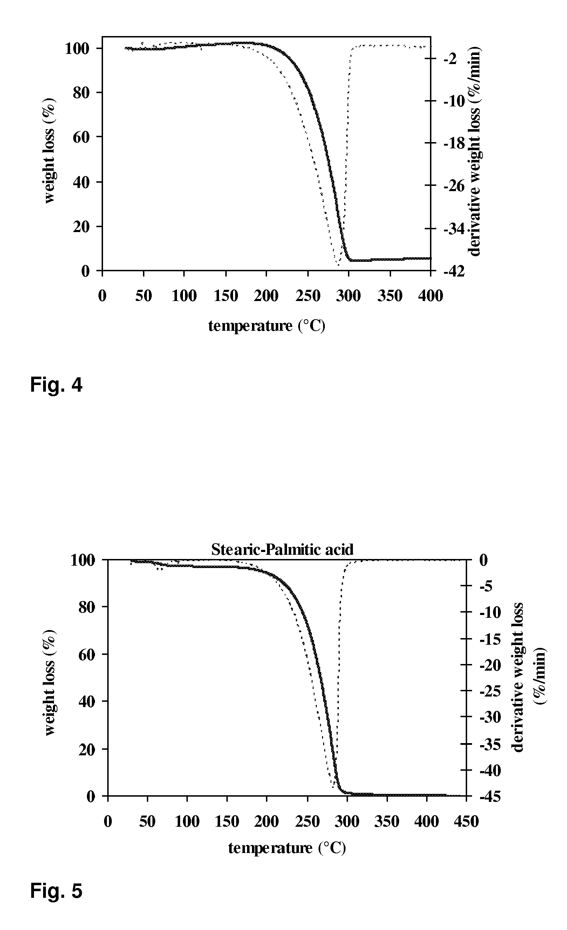

Production of a dry powder consisting of Sodium Ibandronate (Na-IBAN) - containing micro particles:
A concentrated aqueous solution of sodium Ibendronate (Na-IBAN) (about 285 mg/ml) was mixed with molten stearic acid (SA) with a ratio Na IBAN to SPA of 1:9 in the feed vessel at a pressure of 4 bar and a temperature of 70°C, forming an emulsion. The water content of the emulsion was ca. 26 weight %. After spraying using liquefied carbon dioxide at a pressure of 60 bar, a dry, pourable micronized powder was formed with a water content of less than 0.5 Weight-%. TGA (thermogravimetric analysis) shows that the residual water in the powder is about 0.5% w/w, as measurable by minimum weight loss between 50 and 100°C. (Fig. 4). Water removed by the process was about 98% of total amount of water originally added to the mixture.

### Example 3:

Production of a dry powder consisting of Metoclopramide-Hydrochloride (MCP-HCl) containing micro particles:
A mixture of molten Polyethylenglycole 6000 (PEG 6000) and concentrated aqueous solution of MCP-HCl (750 mg/ml) with PEG : MCP HCl ratio of 85:15 % weight was added to the feed vessel at a pressure of 4 bar and a temperature of 70°C. The water content of the mixture was ca. 16 weight-%. After spraying using liquefied carbon dioxide at a pressure of 55 bar, a dry, pourable powder was formed with a water content of less than 5 weight-%.

### Example 4:

Production of a dry powder consisting of Metoclopramide-Hydrochloride (MCP-HCl) containing micro particles:
An emulsion comprising a mixture of 90 weight.-% cetearyl alcohol and 10 weight -% Compritol 888 ATO (in following: CC) and MCP-HCl aqueous solution at 750 mg/ml, with ratio m(CC): m(MCP-HCl) = 85:15 was added to the feed vessel at a pressure of 4 bar and a temperature of 75°C. The water content of the emulsion was ca. 15 weight-%. After spraying using liquefied carbon dioxide at a pressure of 55 bar a dry, flowable, pourable micronized powder with a low water content was formed.

### Example 5:

Production of a micronized dry powder consisting of stearic palmitic acid (SPA) - micro particles:
In a number of experiments, SPA was mixed with different amount of water in the mixing vessel at a pressure of 3,5 bar and a temperature of 75°C using different mixing weight ratios (SPA:(H₂O) = 26:5 and 25:10), forming an emulsion.

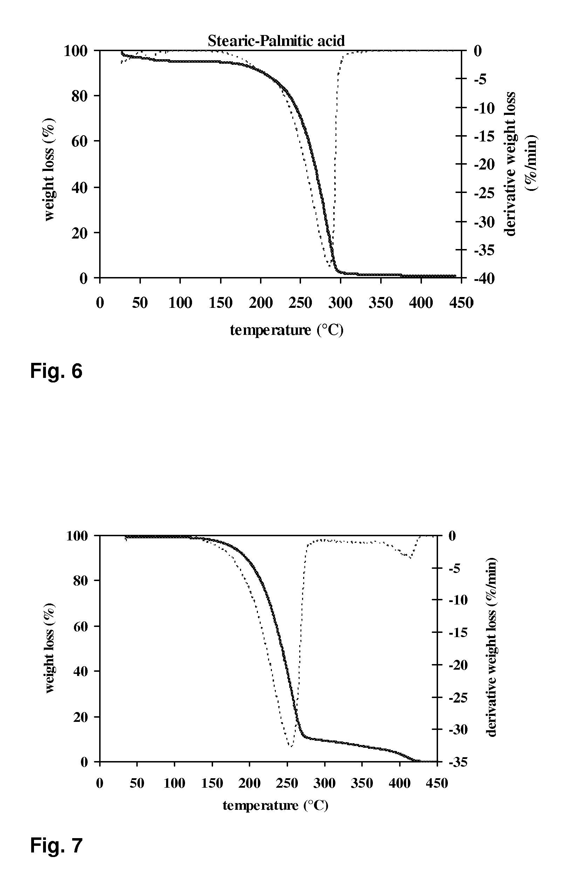

After spraying, using liquefied carbon dioxide at a pressure of 64 bar, a dry, freely flowable, powder was formed. The residual water content was less than 2,5 weight-%, in the first (26:5) mixture, and 3,8 weight-% in the second (25:10), mixture. The amount of water removed during the process was 87% and 90.5%, respectively, as shown in TGA plots (Fig. 5 and 6).

### Example 6

Production of a micronized dry powder (microparticles) consisting of a blend of excipients with different physico-chemical properties:
In two different experiments, cetearyl alcohol (CA) and Compritol 888 ATO (C) were melted at 90:10 w/w ratio, and subsequently emulsified with different amounts of water in the mixing vessel at a pressure of 4 bar and a temperature of 70°C. The water added to the excipients blend was 19.4% and 28.6% w/w.

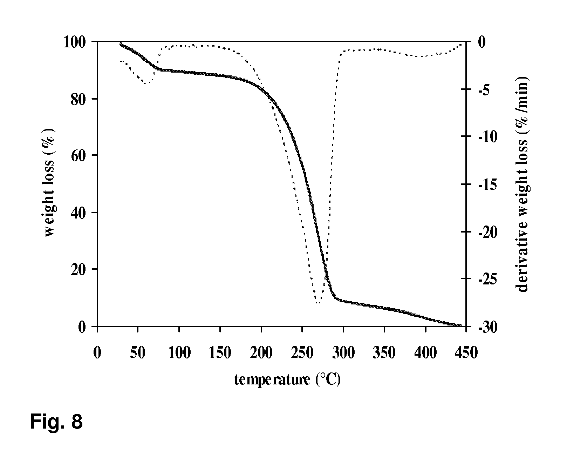

After spraying, using liquefied carbon dioxide at a pressure of 63 bar, a dry, freely flowable, powder was formed. The residual water content, measured by TGA, was less than 1% in and 9.9 % w/w, respective to the 19.4% and 28.6% original amount present in the emulsion before spraying. The amount of water removed during the process was 95% and 65% respectively, as shown in TGA plots (Fig. 7 and 8).

### Reference list

1. Device
2. Pressure vessel
3. Two component nozzle
4. Line
5. Line
6. Feed vessel
7. Mixing device
8. Heater
9. Nozzle orifice
10. Heating device

## Claims

1. Process for production of dry powdery products, **characterized in that** a feed substance or a mixture of feed substances is mixed with water, forming a water-containing mixture of a water content of up to 50 weight-%,
the water containing mixture and supercritical or liquid pressurized carbon dioxide is fed simultaneously to the orifice of a two-component nozzle (3), the supercritical or liquid pressurized carbon dioxide is released and depressurized, forming gaseous carbon dioxide and carbon dioxide snow, the water-containing mixture is brought into contact with the releasing and depressurizing carbon dioxide, whereby an atomisation and drying of the mixture is achieved, whereby the water containing mixture is heated before or during it is fed to the two component nozzle (3) such that the temperature of the water containing mixture at the time of spraying is between 60°C and 95°C.

2. Process according to claim 1, **characterized in that** the feed substance or the mixture of feed substances is pressurized before it is fed to the nozzle (3).

3. Process according to claim 2, **characterized in that** the feed substance or the mixture is pressurized to a pressure of about 2 to 10 bar.

4. Process according to one of the previous claims, **characterized in that** the released supercritical or liquid pressurized carbon dioxide is depressurized to a value of 1 -2 bar.

5. Process according to one of the previous claims, **characterized in that** at least one of the feed substances is a hydrophilic substance which is dissolved or dispersed in the water.

6. Process according to claim 5, **characterized in that** the hydrophilic substance is pharmaceutically active agent, or a pharmaceutical excipient.

7. Process according to one of the previous claims, **characterized in that** at least one or more lipophilic or amphiphilic excipient is fed to the solution or dispersion of the hydrophilic substance, forming an emulsion thereof.

8. Process according to one of the previous claims, **characterized in that** at least one of the feed substances contains a biphosphonate, a salt thereof, a GABA modulator agent, a protein, a peptide and/or an bioactive substance.

9. Process according to one of the previous claims, **characterized in that** at least one of the feed substances contains Ibandronate, particularly Sodium-Ibandronate, Metoclopramide, Pregabalin, cetearyl alcohol, glyceryl behenate and/or stearic palmitic acid.

10. Process according to one of the previous claims, **characterized in that** at least one of the feed substances comprises fatty alcohols, fatty acids, fatty esters and their derivatives as an excipient, or a mixture thereof.

11. Process according to one of the previous claims, **characterized in that** at least one of the feed substances comprises polyethylene glycols (PEG), poloxamers and PEG-fatty acids or lipidic derivatives, alone or mixed with fatty or polymeric.

## Patentansprüche

1. Verfahren zum Herstellen von trockenen pulverförmigen Produkten, **dadurch gekennzeichnet, dass** ein Zufuhrstoff oder ein Gemisch von Zufuhrstoffen mit Wasser gemischt wird, um ein wasserhaltiges Gemisch mit einem Wassergehalt von bis zu 50 Gew.-% zu bilden, das wasserhaltige Gemisch und überkritisches oder flüssiges druckbeaufschlagtes Kohlendioxid gleichzeitig der Öffnung einer Zweikomponentendüse (3) zugeführt werden, das überkritische oder flüssige druckbeaufschlagte Kohlendioxid freigegeben und sein Druck verringert wird, um gasförmiges Kohlendioxid und Kohlendioxidschnee zu bilden, das wasserhaltige Gemisch mit dem freigegebenen und seinen Druck verringernden Kohlendioxid in Kontakt gebracht wird, wodurch Zerstäuben und Trocknen des Gemischs erzielt wird, wobei das wasserhaltige Gemisch erhitzt wird, bevor oder während es der Zweikomponentendüse (3) zugeführt wird, so dass die Temperatur des wasserhaltigen Gemischs zum Zeitpunkt des Versprühens zwischen 60 °C und 95 °C beträgt.

2. Verfahren gemäß Anspruch 1, **dadurch gekennzeichnet, dass** der Zufuhrstoff oder das Gemisch von Zufuhrstoffen druckbeaufschlagt wird, bevor er/es der Düse (3) zugeführt wird.

3. Verfahren gemäß Anspruch 2, **dadurch gekennzeichnet, dass** der Zufuhrstoff oder das Gemisch auf einen Druck von 2 bis 10 bar druckbeaufschlagt wird.

4. Verfahren gemäß einem der vorstehenden Ansprüche, **dadurch gekennzeichnet, dass** der Druck des freigegebenen überkritischen oder flüssigen druckbeaufschlagten Kohlendioxids auf einen Wert von 1-2 bar gesenkt wird.

5. Verfahren gemäß einem der vorstehenden Ansprüche, **dadurch gekennzeichnet, dass** wenigstens einer der Zufuhrstoffe ein hydrophiler Stoff ist, der in dem Wasser gelöst oder dispergiert wird.

6. Verfahren gemäß Anspruch 5, **dadurch gekennzeichnet, dass** der hydrophile Stoff ein pharmazeutischer Wirkstoff oder ein pharmazeutischer Hilfsstoff ist.

7. Verfahren gemäß einem der vorstehenden Ansprüche, **dadurch gekennzeichnet, dass** der Lösung oder Dispersion des hydrophilen Stoffs wenigstens ein oder mehrere lipophile oder amphiphile Hilfsstoffe zugeführt werden, um eine Emulsion davon zu bilden.

8. Verfahren gemäß einem der vorstehenden Ansprüche, **dadurch gekennzeichnet, dass** wenigstens einer der Zufuhrstoffe ein Biphosphonat, ein Salz davon, ein GABA-Modulatormittel, ein Protein, ein Peptid und/oder einen bioaktiven Stoff umfasst.

9. Verfahren gemäß einem der vorstehenden Ansprüche, **dadurch gekennzeichnet, dass** wenigstens einer der Zufuhrstoffe Ibandronat, insbesondere Natriumibandronat, Metoclopramid, Pregabalin, Ceterarylalkohol, Glycerylbehenat und/oder Stearinpalmitinsäure enthält.

10. Verfahren gemäß einem der vorstehenden Ansprüche, **dadurch gekennzeichnet, dass** wenigstens einer der Zufuhrstoffe Fettalkohole, Fettsäuren, Fettester und deren Derivate als einen Hilfsstoff oder ein Gemisch davon umfasst.

11. Verfahren gemäß einem der vorstehenden Ansprüche, **dadurch gekennzeichnet, dass** wenigstens einer der Zufuhrstoffe Polyethylenglycole (PEG), Poloxamere und PEG-Fettsäuren oder Lipidderivate allein oder gemischt mit Fett oder Polymer umfasst.

## Revendications

1. Procédé de production de produits pulvérulents secs, **caractérisé en ce qu'**une substance de départ ou un mélange de substances de départ sont mélangés avec de l'eau, formant un mélange contenant de l'eau de teneur en eau allant jusqu'à 50 % en masse,
le mélange contenant de l'eau et le dioxyde de carbone pressurisé liquide ou supercritique sont introduits simultanément dans l'orifice d'une buse bicomposant (3), le dioxyde de carbone pressurisé liquide ou supercritique est libéré et dépressurisé, formant du dioxyde de carbone gazeux et de la neige carbonique, le mélange contenant de l'eau est mis en contact avec le dioxyde de carbone en cours de libération et de dépressurisation, ce qui permet d'obtenir une atomisation et un séchage du mélange, le mélange contenant de l'eau étant chauffé avant ou après introduction dans la buse bicomposant (3) de sorte que la température du mélange contenant de l'eau au moment de l'atomisation soit comprise entre 60 °C et 95 °C.

2. Procédé selon la revendication 1, **caractérisé en ce que** la substance de départ ou le mélange de substances de départ sont pressurisés avant introduction dans la buse (3).

3. Procédé selon la revendication 2, **caractérisé en ce que** la substance de départ ou le mélange sont pressurisés jusqu'à une pression d'environ 2 à 10 bars.

4. Procédé selon l'une des revendications précédentes, **caractérisé en ce que** le dioxyde de carbone pressurisé liquide ou supercritique libéré est dépressurisé jusqu'à une valeur de 1 à 2 bars.

5. Procédé selon l'une des revendications précédentes, **caractérisé en ce qu'**au moins une des substances de départ est une substance hydrophile qui est dissoute ou dispersée dans l'eau.

6. Procédé selon la revendication 5, **caractérisé en ce que** la substance hydrophile est un principe actif pharmaceutique ou un excipient pharmaceutique.

7. Procédé selon l'une des revendications précédentes, **caractérisé en ce qu'**au moins un plus des excipients lipophiles ou amphiphiles est introduit dans la solution ou la dispersion de la substance hydrophile, formant une émulsion de celle-ci.

8. Procédé selon l'une des revendications précédentes, **caractérisé en ce qu'**au moins l'une des substances de départ contient un biphosphonate, un sel de celui-ci, un agent modulateur de GABA, une protéine, un peptide et/ou une substance bioactive.

9. Procédé selon l'une des revendications précédentes, **caractérisé en ce qu'**au moins l'une des substances de départ contient de l'Ibandronate, en particulier de l'Ibandronate de sodium, du Métoclopramide, de la Prégabaline, de l'alcool cétoarylique, du béhénate de glycéryle et/ou de l'acide palmitique stéarique.

10. Procédé selon l'une des revendications précédentes, **caractérisé en ce qu'**au moins l'une des substances de départ comprend des alcools gras, des acides gras, des esters gras et leurs dérivés en tant qu'excipients ou mélanges de ceux-ci.

11. Procédé selon l'une des revendications précédentes, **caractérisé en ce qu'**au moins l'une des substances de départ comprend des polyéthylène glycols (PEG), des poloxamères et des PEG-acides gras ou des dérivés lipidiques, seuls ou en mélange avec des substances grasses ou des polymères.
